(19) Europäisches Patentamt
European Patent Office
Office européen des brevets

(11) Veröffentlichungsnummer: **0 275 832 B1**

# (12) EUROPÄISCHE PATENTSCHRIFT

(45) Veröffentlichungstag der Patentschrift: **16.10.91**

(51) Int. Cl.5: **C07C 323/07**, C07C 323/16, C07C 319/00

(21) Anmeldenummer: **87810766.3**

(22) Anmeldetag: **18.12.87**

## (54) Verfahren zur Herstellung von Merkaptomethylphenolen.

(30) Priorität: **24.12.86 CH 5237/86**

(43) Veröffentlichungstag der Anmeldung: **27.07.88 Patentblatt 88/30**

(45) Bekanntmachung des Hinweises auf die Patenterteilung: **16.10.91 Patentblatt 91/42**

(84) Benannte Vertragsstaaten: **BE DE ES FR GB IT NL SE**

(56) Entgegenhaltungen:
**EP-A- 0 165 209**
**GB-A- 1 184 533**

**CHEMICAL ABSTRACTS, Band 83, Nr. 10, 8. September 1975, Seite 20, Spalte 1, Zusammenfassungsnr. 79881h, Columbus, Ohio, US; F.A. ABDULLAEVA "Synthesis of screened phenols", & AZERB. KHIM ZH. 1973, (5-6), 66-9**

**JOURNAL OF THE CHEMICAL SOCIETY, 14. April 1954, Seiten 1124-1130, Burlington House, W.I., London, GB; F. POPPELSDORF et al.: "Reactions of Thiols and Thiothers. Part I. An Analogue of the Mannich Reaction involving Thiols, Formaldehyde, and Active Methylene or Methylidyne Compounds"**

(73) Patentinhaber: **CIBA-GEIGY AG**
**Klybeckstrasse 141**
**CH-4002 Basel(CH)**

(72) Erfinder: **Stegmann, Werner, Dr.**
**Unter der Sonnhalde 9**
**CH-4410 Liestal(CH)**
Erfinder: **Meier, Hans Rudolf, Dr.**
**Rte du Confin 54**
**CH-1723 Marly(CH)**
Erfinder: **Evans, Samuel, Dr.**
**Route des Charbonnières 1**
**CH-1723 Marly(CH)**
Erfinder: **Martin, Roger**
**Uf em Bärg 2**
**CH-1711 Tentlingen(CH)**
Erfinder: **Luisoli, Reto**
**Stutzring 3**
**CH-4434 Hölstein(CH)**

EP 0 275 832 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Merkaptomethylphenolen aus Phenolen durch Umsetzung mit Formaldehyd und Merkaptanen.

Es ist bekannt, dass man Aryl- oder Alkylthiomethylnaphthole durch Umsetzung z.B. von $\beta$-Naphthol mit Formaldehyd und Aryl- oder Alkylmerkaptan erhält, wenn gleichzeitig Triethylamin anwesend ist (vgl. F. Poppelsdorf et al., J. Chem. Soc. 1954, 1124 ff). Zur Erzielung befriedigender Ausbeuten sind jedoch Reaktionszeiten von ungefähr 6 Tagen erforderlich.

Es ist ferner in der EP-A-0 165 209 erwähnt, 2,4-Merkaptomethylphenole beispielsweise aus Phenolen mit Formaldehyd und Merkaptanen in Gegenwart von Dialklyl- oder Trialkylaminen herzustellen. Spezifisch wird jedoch nur Dibutylamin eingesetzt.

Da Merkaptomethylphenole wertwolle Antioxidantien sind, besteht weiterhin das Bedürfnis nach einer Verfahrensverbesserung. Es wurde nun gefunden, dass Merkaptomethylphenole in hoher Ausbeute und Reinheit in vorteilhaft kurzen Reaktionszeiten erhalten werden, wenn die Umsetzung in Gegenwart eines Methyl- oder Ethylamins erfolgt.

Gegenstand vorliegender Erfindung ist daher ein Verfahren zur Herstellung von Verbindungen der Formeln I oder II

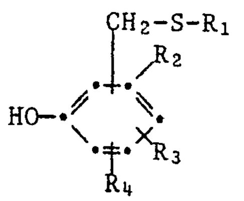

(I)

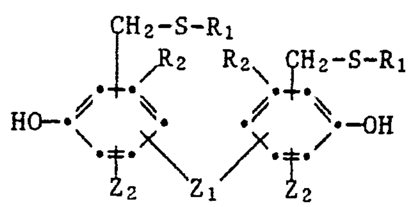

(II)

,

worin

$R_1$ $C_1$-$C_{20}$-Alkyl bedeutet, welches unsubstituiert ist oder durch 1 oder 2 Hydroxylgruppen substituiert oder durch -O- unterbrochen ist, oder $C_1$-$C_4$-Alkylen-$COOR_5$, $C_1$-$C_4$-Alkylen-CO-$NR_5R_6$, $C_5$-$C_{12}$-Cycloalkyl, Phenyl, 1-Naphthyl, 2-Naphthyl, $C_1$-$C_4$-Alkyl-Phenyl oder Phenyl-$C_1$-$C_4$-Alkyl bedeutet,

$R_2$ Wasserstoff, $C_1$-$C_{20}$-Alkyl, $C_2$-$C_{18}$-Alkenyl oder Halogen ist,

$R_3$ und $R_4$ unabhängig voneinander $C_1$-$C_{20}$-Alkyl, Allyl, $C_5$-$C_{12}$-Cycloalkyl, Phenyl, Phenyl-$C_1$-$C_4$-Alkyl, Halogen oder -$CH_2$-S-$R_1$ bedeuten, mit der Massgabe, dass mindestens eines von $R_3$ und $R_4$ für -$CH_2$-S-$R_1$ steht,

$R_5$ $C_1$-$C_{20}$-Alkyl, Allyl, $C_5$-$C_{12}$-Cycloalkyl, Phenyl oder Benzyl bedeutet,

$R_6$ Wasserstoff, $C_1$-$C_{20}$-Alkyl oder $C_2$-$C_{18}$-Alkenyl ist,

$Z_1$ für -S- oder -C($Z_3$)($Z_4$)- steht,

$Z_2$ Wasserstoff, $C_1$-$C_{20}$-Alkyl oder -$CH_2$-S-$R_1$ ist,

$Z_3$ Wasserstoff oder Methyl bedeutet und $Z_4$ Wasserstoff oder $C_1$-$C_8$-Alkyl ist, mit der Massgabe, dass die Phenole der Formeln I und II in m-Stellung frei von der funktionellen Gruppe -$CH_2$-S-$R_1$ sind, durch Umsetzung eines Phenols der Formeln III oder IV

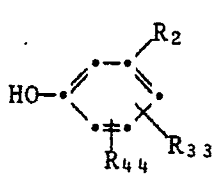

(III)

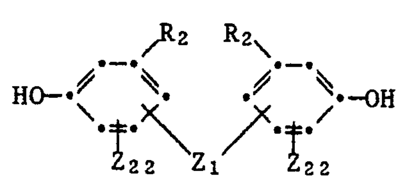

(IV)

,

worin

$R_2$ and $Z_1$ die zuvor genannte Bedeutung haben,

$R_{33}$ und $R_{44}$ unabhängig voneinander Wasserstoff, $C_1$-$C_{20}$-Alkyl, Allyl, $C_5$-$C_{12}$-Cycloalkyl, Phenyl, Phenyl-$C_1$-$C_4$-Alkyl oder Halogen bedeuten, mit der Massgabe, dass mindestens eines von $R_{33}$ und $R_{44}$ für Wasserstoff steht, und

$Z_{22}$ Wasserstoff oder $C_1$-$C_{20}$-Alkyl ist,
mit Formaldehyd oder einer unter den Reaktionsbedingungen Formaldehyd freisetzenden Verbindung und mit mindestens einem Merkaptan $R_1$-SH in Gegenwart einer Base, dadurch gekennzeichnet, dass die Base Mono-, Di-oder Trimethylamin oder Mono- oder Diethylamin ist.

Bevorzugt werden nach dem erfindungsgemässen Verfahren Merkaptomethylphenole der Formel I hergestellt, worin $R_1$ $C_8$-$C_{12}$-Alkyl, -$CH_2$-COO-Alkyl mit 1 bis 18 C-Atomen im Alkylrest, -$CH_2CH_2$-OH, Phenyl oder Benzyl bedeutet, und insbesondere solche, worin $R_1$ $C_8$-$C_{12}$-Alkyl oder -$CH_2$-COO-$CH_2$-C-($C_2H_5$)-$(CH_2)_3$-$CH_3$ ist.

Besonders bevorzugt werden Merkaptomethylphenole hergestellt, die der Formel

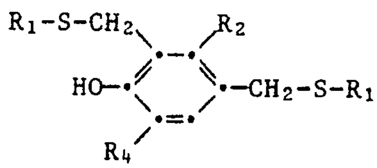

entsprechen,
worin $R_1$, $R_2$ und $R_4$ die zuvor genannte Bedeutung haben, und insbesondere solche, worin $R_2$ Wasserstoff oder Methyl ist und $R_4$ Methyl, t-Butyl oder Cyclohexyl bedeutet.

Ebenfalls von Interesse sind nach dem erfindungsgemässen Verfahren hergestellte Merkaptomethylphenole der Formel

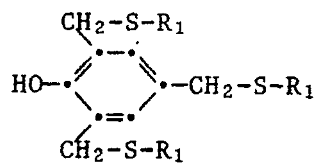

worin $R_1$ die zuvor genannte Bedeutung hat.

$R_1$, $R_2$, $R_3$, $R_4$, $R_5$, $R_6$, $R_{33}$, $R_{44}$, $Z_2$ and $Z_{22}$ bedeuten als $C_1$-$C_{20}$-Alkyl beispielsweise Methyl, Ethyl, n-Propyl, Isopropyl, n-Butyl, sek-Butyl, t-Butyl, n-Pentyl, Isopentyl, n-Hexyl, 1,1-Dimethylbutyl, n-Heptyl, n-Octyl, 2-Ethylhexyl, n-Nonyl, n-Decyl, 1,1,3,3-Tetramethylbutyl, 1,1,3,3-Tetramethylhexyl, n-Undecyl, n-Dodecyl, 1,1,3,3,5,5-Hexamethylhexyl, 2,2,4,6,6-Pentamethylept-4-yl, n-Tetradecyl, n-Hexadecyl, n-Octadecyl oder n-Eicosyl.

$Z_4$ als $C_1$-$C_8$-Alkyl bedeutet beispielsweise Methyl, Ethyl, n-Propyl, Isopropyl, n-Butyl, sek-Butyl, n-Pentyl, Isopentyl, n-Hexyl, 1,1-Dimethylbutyl, n-Heptyl oder n-Octyl, bevorzugt $C_1$-$C_4$-Alkyl und insbesondere Methyl.

$R_2$, $R_3$, $R_4$, $R_{33}$, $R_{44}$, $Z_2$ und $Z_{22}$ sind als Alkyl vorzugsweise $C_1$-$C_4$-Alkyl und bedeuten dann beispielsweise Methyl, Ethyl, n-Propyl, Isopropyl, n-Butyl oder t-Butyl.$R_4$ und $R_{44}$ bedeuten besonders bevorzugt Methyl oder t-Butyl.

$R_1$ ist vorzugsweise $C_2$-$C_{18}$-Alkyl und insbesondere $C_8$-$C_{12}$-Alkyl, wie beispielsweise n-Octyl, 1,1,3,3-Tetramethylbutyl, n-Dodecyl oder t-Dodecyl [ein Gemisch aus 1,1,3,3,5,5-Hexamethylhexyl und 1,1,4,4,6,6,-Pentamethylhept-(4)-yl].

$R_1$ als durch ein oder zwei Hydroxylgruppen substituiertes $C_1$-$C_{20}$-Alkyl bedeutet beispielsweise Hydroxymethyl, 2-Hydroxyethyl, 2-Hydroxypropyl, ,2-Hydroxybutyl, 2-Hydroxyhexyl, 2-Hydroxyoctyl, 2-Hydroxydecyl, 2-Hydroxydodecyl, 2-Hydroxytetradecyl, 2-Hydroxyhexadexyl, 2-Hydroxyoctadecyl, 2-Hydroxyeicosyl oder 2,3-Dihydroxypropyl. Bevorzugt wird $C_1$-$C_4$-Hydroxyalkyl, z.B. 2-Hydroxypropyl oder 2,3-Dihydroxypropyl und insbesondere 2-Hydroxyethyl.

$R_1$ als durch -O- unterbrochenes Alkyl kann eine oder mehrere, z.B. 1 bis 6, insbesondere 1 oder 2 -0-Unterbrechungen aufweisen und bedeutet beispielsweise 3-Oxapropyl, 3-Oxabutyl, 3-Oxapentyl, 3,6-Dioxaheptyl, 3,6,9-Trioxadecyl oder 3,6,9,12,15,18-Hexaoxanonadecyl.

$R_2$ und $R_6$ als $C_2$-$C_{18}$-Alkenyl bedeuten beispielsweise Vinyl, Allyl, But-3-enyl, Pent-4-enyl, Hex-5-enyl, Oct-7-enyl, Dec-9-enyl, Dodec-11-enyl oder Octadec-17-enyl. Bevorzugt wird Vinyl oder Allyl.

$R_1$, $R_3$, $R_4$, $R_5$, $R_{33}$ und $R_{44}$ als $C_5$-$C_{12}$-Cycloalkyl bedeuten beispielsweise Cyclopentyl, Cyclohexyl,

Cycloheptyl, Cyclooctyl, Cyclononyl, Cyclodecyl, Cycloundecyl oder Cyclododecyl. Bevorzugt werden $C_5$-$C_7$-Cycloalkyl und ganz besonders Cyclohexyl.

$R_1$, $R_3$, $R_4$, $R_{33}$ und $R_{44}$ als Phenyl-$C_1$-$C_4$-Alkyl bedeuten beispielsweise Benzyl, Phenylethyl, α-Methylbenzyl oder α,α-Dimethylbenzyl. Bevorzugt wird Benzyl.

$R_1$ als $C_1$-$C_4$-Alkyl-Phenyl kann z.B. 1 bis 3, vor allem 1 oder 2 Alkylgruppen aufweisen und bedeutet beispielsweise o-, m- oder p-Tolyl, ,2,4-, 2,6-, 3,5- oder 3,6-Dimethylphenyl, Ethylphenyl, Propylphenyl, Isopropylphenyl, t-Butylphenyl oder Di-t-butylphenyl. Bevorzugt wird Tolyl.

$R_2$, $R_3$, $R_4$, $R_{33}$ und $R_{44}$ als Halogen bedeuten beispielsweise Chlor oder Brom, insbesondere Chlor.

Im erfindungsgemässen Verfahren können die Reaktanden Phenol, Formaldehyd und Merkaptan in stöchiometrischen Mengen eingesetzt werden. Mitunter kann es aber von Vorteil sein, wenn der Formaldehyd und/oder das Merkaptan im Ueberschuss eingesetzt werden. Es können auch Gemische von Phenolen und/oder Merkaptanen umgesetzt werden.

Das erfindungsgemässe Verfahren wird in Gegenwart von Mono-, Di-oder Trimethylamin oder Mono- oder Diethylamin als Base durchgeführt. Bevorzugt wird Mono- oder Dimethylamin und insbesondere Dimethylamin eingesetzt.

Die Base kann z.B. in Form einer 10-35 %-ige Lösung in Ethanol, Methanol oder anderen niederen Alkoholen oder in reiner Form verwendet werden. Dimethylamin kann auch als Gas eingesetzt werden.

Im erfindungsgemässen Verfahren kann die Base beispielsweise in einer Menge von 1-50 Mol%, vorzugsweise 2-30 Mol% und insbesondere 5-20 Mol%, bezogen auf das Merkaptan, eingesetzt werden.

Das erfindungsgemässe Verfahren kann in Anwesenheit eines Lösungsmittels durchgeführt werden.

Geeignete Lösungsmittel sind z.B. Alkohole mit ein bis sechs Kohlenstoffatomen, wie beispielsweise Methanol, Ethanol, Propanol, Butanol, Pentanol oder Hexanol. Man kann aber auch Diole, Polyole sowie deren Ether verwenden, wie beispielsweise Glykol, Glycerin, Polyethylenglykol. Die Reaktion lässt sich auch in polaren aprotischen Lösungsmitteln, wie beispielsweise Dimethylformamid oder Dimethylsulfoxid durchführen, oder es können hochsiedende aromatische oder aliphatische, gegebenenfalls chlorierte Kohlenwasserstoffe wie beispielsweise Toluol, Ligroin oder Chlorbenzol eingesetzt werden. Bevorzugt wird Dimethylformamid verwendet, welches gegebenenfalls mit einem der zuvor genannten niederen Alkohole oder chlorierten Kohlenwasserstoffe verdünnt ist.

Bevorzugt wird jedoch das erfindungsgemässe Verfahren in Abwesenheit eines Lösungsmittels durchgeführt.

Das erfindungsgemässe Verfahren kann vorteilhafterweise bei Temperaturen von 80-160 °C, bevorzugt von 90-150 °C und insbesondere von 90-130 °C und gegebenenfalls unter Druck (z.B. 0,01 bis 5 bar) durchgeführt werden. In Abwesenheit eines Lösungsmittels wird die Umsetzung bevorzugt unter Ueberdruck ausgeführt.

Die Reaktionszeiten können je nach spezifischem Phenol und Merkaptan schwanken und betragen z.B. 1 bis 24, insbesondere 1 bis 6 Stunden. Das Reaktionsgemisch wird zweckmässig in einer Stickstoffatmosphäre unter Rückfluss erhitzt.

Nach dem Abkühlen auf Raumtemperatur wird das Reaktionsgemisch nach den üblichen Trenn- und Reinigungsmethoden aufgearbeitet.

Es ist jedoch ein weiterer Vorteil des erfindungsgemässen Verfahrens, dass die Endprodukte in einer Reinheit anfallen, die für viele Zwecke ihre direkte Weiterverwendung erlaubt.

Die nach dem erfindungsgemässen Verfahren hergestellten Merkaptomethylphenole sind zum grössten Teil bekannte Verbindungen.

Die als Ausgangsstoffe dienenden Phenole und Merkaptane sind ebenfalls bekannt, sind teilweise im Handel erhältlich oder können nach bekannten Methoden hergestellt werden.

Für die erfindungsgemässe Umsetzung wird Formaldehyd oder eine letzteres unter den Reaktionsbedingungen abgebende Verbindung, wie z.B. Paraformaldehyd oder Hexamethylentetramin eingesetzt. Bevorzugt wird Formaledehyd und insbesondere Paraformaldehyd verwendet.

Die nach dem erfindungsgemässen Verfahren hergestellten Verbindungen der Formeln I und II können als Stabilisatoren für organisches Material gegen dessen Schädigung durch Einwirkung von Sauerstoff, Wärme, Licht oder energiereiche Strahlung verwendet werden.

Bevorzugt ist die Verwendung der Verbindungen als Antioxidantien in organischen Polymeren und in Elastomeren, oder die Verwendung in Mineralölen oder synthetischen Schmierstoffen, wie z.B. in der EP-A-0 165 209 beschrieben.

Die Stabilisatoren werden den Kunststoffen bzw. den Schmiermitteln im allgemeinen in einer Konzentration von 0,01-10 Gew.-%, berechnet auf das zu stabilisierende Material, zugesetzt. Vorzugsweise werden 0,05 bis 5,0 Gew.-%, besonders bevorzugt 0,1 bis 2,0 Gew.-% der Verbindungen, berechnet auf das zu stabilisierende Material, in dieses eingearbeitet.

Die Einarbeitung kann beispielsweise durch Einmischen der Verbindungen der Formeln I und II und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die erfindungsgemäss hergestellten Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetzbarem Polyethylen werden die Verbindungen vor der Vernetzung beigefügt.

In der Praxis können die Merkaptomethylphenole der Formeln I und II zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z.B. weitere Antioxidantien, Metallpassivatoren, Rostinhibitoren, Viskositäts-Index Verbesserer, Stockpunkterniedriger, Dispergiermittel/Tenside und Verschleissschutzadditive.

Die folgenden Beispiele erläutern die Erfindung weiter. Die Reinheit der hergestellten Verbindung wird mittels "high pressure liquid chromatography" bestimmt. Aus den Rohausbeuten und der Reinheit lässt sich eine absolute Ausbeute berechnen, die jeweils in Klammern nach der Reinheit aufgeführt ist.

Beispiel 1: Herstellung von 2,4-Bis(n-octylthiomethyl)-6-methyl phenol

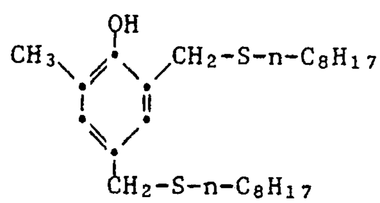

Ein Gemisch von 16,22 g (0,15 Mol) o-Kresol, 43,88 g (0,3 Mol) n-Octanthiol, 18,02 g (0,6 Mol) Paraformaldehyd, 4,1 g 33 %-iges ethanolisches Dimethylamin (entspricht etwa 0,03 Mol Dimethylamin) und 22,5 ml (21,35 g) N,N-Dimethylformamid werden in einem Sulfierkolben mit Rückflusskühler und mechanischem Rührer unter Stickstoff während 2 Stunden unter Rückfluss erhitzt. Die Innentemperatur (IT) beträgt 115 °C. Anschliessend werden bei einer Badtemperatur von 90-95 °C und reduziertem Druck die flüchtigen Bestandteile abdestilliert. Der Rückstand wird anschliessend bei 100-120 °C und 1,33 mbar getrocknet. Man erhält 61,8 g (96,9 % der Theorie) 2,4-Bis(n-octylthiomethyl)-6-methylphenol als gelbliche Flüssigkeit, mit einer Reinheit von 94,6 % (absolute Ausbeute: 91,7%).

Analysewerte:

| | | | |
|---|---|---|---|
| Berechnet: | 70,70 % C | Gefunden: | 70,71 % C |
| | 10,44 % H | | 10,41 % H |
| | 15,10 % S | | 15,09 % S |

In den nachfolgenden Beispielen 2 und 3 werden Verfahrensvarianten für die Herstellung von 2,4-Bis(n-octyl-thiomethyl)-6-methylphenol in Gegenwart eines Lösungsmittels und in den Beispielen 4 und 5 in Abwesenheit eines Lösungsmittels beschrieben.

Beispiel 2:

Ein Gemisch von 108,14 g (1 Mol) o-Kresol, 292,56 g (2 Mol) n-Octanthiol, 120,12 g (4 Mol) Paraformaldehyd, 27,33 g 33 %-igem ethanolischem Dimethylamin (entspricht etwa 0,2 Mol Dimethylamin) und 50 ml (47,4 g) N,N-Dimethylformamid werden analog zu Beispiel 1 während 2 Stunden unter Rückfluss erhitzt (IT = 115 °C).

Nach Aufarbeitung wie in Beispiel 1 wird das Produkt noch wie folgt gereinigt: das Rohprodukt wird in 800 ml Hexan aufgenommen und in einem Scheidetrichter mit 4 x 200 ml Wasser gewaschen. Die Hexanphase wird dann abgetrennt und getrocknet. Nach destillativer Entfernung des Lösungsmittels erhält man 409,5 g (96,4 % der Theorie) einer gelblichen Flüssigkeit, die $^1$H-NMR-spektroskopisch und dünnschichtchromatographisch mit der unter Beispiel 1 erhaltenen Verbindung identisch ist und eine Reinheit von 97,9 % aufweist (absolute Ausbeute: 94,4 %).

Beispiel 3:

Man geht wie in Beispiel 1 vor, jedoch unter Verwendung folgenden Gemisches: 16,22 g (0,15 Mol) o-Kresol, 43,88 g (0,3 Mol) n-Octanthiol, 18,02 g (0,6 Mol) Paraformaldehyd, 22,5 ml (21,35 g) N,N-Dimethylformamid und 1,14 g (0,031 Mol) Dimethylamin. Das Dimethylamin wird als Gas in die Suspension der zuvor genannten Komponenten (IT der Suspension: 20-30 °C) eingeleitet. Man erhält 55,6 g (87,2 % der Theorie) 2,4-Bis(n-octyl-thiomethyl)-6-methylphenol als gelbliche Flüssigkeit mit einer Reinheit von 98,3 % (absolute Ausbeute: 85,7 %).

Beispiel 4:

Bei Raumtemperatur werden in einer Apparatur, bestehend aus einem 1 l Reaktionsgefäss (bis 2 bar Ueberdruck zugelassen) mit Impellerrührer, Innenthermometer, Destillationsaufsatz mit Kühler und Vorlage mit Vakuumanschluss, Stickstoffbegasung und Gaseinleitungsrohr, nacheinander 194,6 g (1,80 Mol) o-Kresol, 113,5 g (3,78 Mol) Paraformaldehyd (100 %) und 526,7 g (3,60 Mol) 1-Octanthiol vorgelegt. Die Suspension wird mit Stickstoff inertisiert und anschliessend unter starkem Rühren bei Raumtemperatur (25 °C) auf ca. 20 mbar evakuiert. Anschliessend wird durch ein Gaseinleitungsrohr 9,7 g (0,216 Mol) Dimethylamin als Gas innert 10 Minuten in die Suspension eingeleitet. Dabei ist eine deutliche Exothermie zu beobachten, die Innentemperatur steigt dabei etwa um 10 °C, während das Vakuum in der Apparatur auf ca. 100 mbar fällt.

Die Suspension wird auf 120 °C aufgeheizt und 5-6 Stunden lang bei dieser Temperatur gerührt. Der Druck steigt dabei von 100 mbar auf einen Druck von ca. 2,2 bar (entsprechend einem Ueberdruck von 1,2 bar). Die Suspension geht dabei in eine klare gelbbraune Schmelze über, die gegen Ende der Reaktion durch das entstandene Wasser stark trübe wird.

Das Reaktionsgemisch wird auf 50 °C abgekühlt. Bei dieser Temperatur wird durch Anlegen eines Vakuums von 20 mbar ein Gemisch von Dimethylamin, Wasser und etwas überschüssigem Paraformaldehyd abdestilliert, bis bei 100 °C und 20 mbar die Destillation beendet ist.

Die klar gewordene Schmelze wird nun mittels Wasserdampfdestillation (IT ca. 135 °C) und anschliessender Trocknung bei 150 °C und 20 mbar vom überschüssigen Octanthiol befreit.

Man erhält 690 g (90 % der Theorie) 2,4-Bis(n-octylthiomethyl)-6-methylphenol mit einer Reinheit von 89,6 % (absolute Ausbeute: 80,6 %).

Beispiel 5:

Bei Raumtemperatur werden in einer Apparatur, bestehend aus einem 350 ml Sulfierkolben mit Propellerrührer, Gaseinleitungsrohr, Innenthermometer, Stickstoffbegasungsvorrichtung und Sole-gekühltem Rückflusskühler 32,4 g (0,30 Mol) o-Kresol, 87,8 g (0,60 Mol) 1-Octanthiol und 18,9 g (0,63 Mol) Paraformaldehyd vorgelegt. Anschliessend werden 4,06 g (0,09 Mol) Dimethylamin als Gas innert 10 Minuten unter kräftigem Rühren in die Suspension eingeleitet. Dabei steigt die Innentemperatur um ca. 20 °C. Die Suspension wird unter leichter Stickstoffbegasung auf 112-114 °C aufgeheizt. Dabei geht die Suspension in eine anfänglich klare aufgeheizt. Schmelze über, und es setzt Rückfluss ein. Während ca. 4 Stunden wird weiter unter Rückfluss gerührt. Die Schmelze wird zuerst trübe, später zweiphasig (Wasser) und die Innentemperatur sinkt auf 102-104 °C.

Die Aufarbeitung des Reaktionsgemisches erfolgt wie in Beispiel 4.

Man erhält 120,8 g (84,8 % der Theorie) 2,4-Bis(n-octylthiomethyl)-6-methylphenol mit einer Reinheit von 89,9 % (absolute Ausbeute: 76,2 %).

Beispiel 6: Herstellung von 2,4,6,-Tris[3′-(2″-ethylhexyloxycarbonyl)-2′-thiapropyl]-phenol

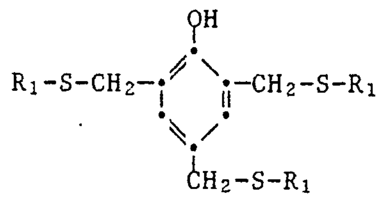

worin

$R_1$ = 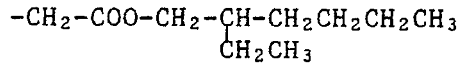

bedeutet.

Ein Gemisch von 18,82 g (0,2 Mol) Phenol, 122,60 g (0,6 Mol) Thioglykolsäure-2-ethylhexylester, 36,0 g (1,2 Mol) Paraformaldehyd, 5,46 33 %-igem ethanolischem Dimethylamin (entspricht etwa 0,04 Mol Dimethylamin) und 2,0 ml (1,9 g) N,N-Dimethylformamid werden in einem Sulfierkolben mit Rückflusskühler und Rührer unter Stickstoff während 6 Stunden auf 90° C erhitzt. Anschliessend werden 200 ml Toluol und 100 ml Wasser zugegeben und kurz aufgerührt. Die organische Phase wird im Scheidetrichter abgetrennt und zur Trockene eingedampft. Der Rückstand wird während 2 Stunden bei 70° C und 0,133 mbar getrocknet. Man erhält 142,7 g (96 % der Theorie) 2,4,6-Tris[3'-(2''-ethylhexyloxycarbonyl)-2'-thiapropyl]-phenol als klares, farbloses Oel mit einer Reinheit von 80,8 % (absolute Ausbeute: 77,6 %).

Analysewerte:

| | | | |
|---|---|---|---|
| Berechnet: | 63,03 % C | Gefunden: | 62,59 % C |
| | 8,95 % H | | 8,98 % H |
| | 12,94 % S | | 12,38 % S |

Beispiel 7:

Man geht wie in Beispiel 6 vor, jedoch unter Verwendung von 1,80 g (0,04 Mol) Dimethylamin-Gas. Das Gas wird bei 20-30° C (Innentemperatur der Suspension) in die Suspension eingeleitet.

Man erhält 141,2 g (95 % der Theorie) eines klaren, farblosen Oels, das $^1$H-NMR-spektroskopisch und dünnschichtchromatographisch mit der unter Beispiel 6 erhaltenen Verbindung identisch ist und eine Reinheit von 82,5 % aufweist (absolute Ausbeute: 78,4 %).

Beispiel 8: Herstellung von 2,6-Bis(n-octylthiomethyl)-4-t-butyl-phenol

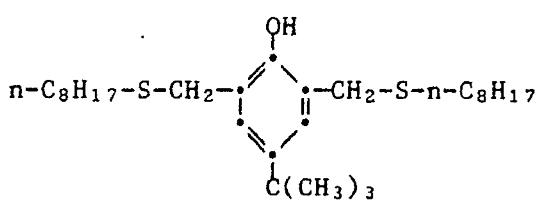

Man geht wie in Beispiel 1 vor, jedoch unter Verwendung folgenden Gemisches: 22,53 g (0,15 Mol) 4-t-Butylphenol, 18,02 g (0,6 Mol) Paraformaldehyd, 43,88 g (0,3 Mol) n-Octanthiol, 4,1 g 33 %-iges ethanolisches Dimethylamin (entspricht etwa 0,03 Mol Dimethylamin) und 22,5 ml (21,4 g) N,N-Dimethylformamid. Die Reaktionszeit beträgt 3 Stunden.

Das Rohprodukt wird in 150 ml Essigsäureethylester aufgenommen und mit 100 ml Wasser gewaschen.

Nach Eindampfen der organischen Phase zur Trockene erhält man 51 g (97 % der Theorie) 2,6-Bis(n-octylthiomethyl)-4-t-butylphenol als farbloses Oel mit einer Reinheit von 93,3 % (absolute Ausbeute: 90,5 %).

Analysewerte:

| | | | |
|---|---|---|---|
| Berechnet: | 72,04 % C | Gefunden: | 71,91 % C |
| | 10,80 % H | | 10,83 % H |
| | 13,74 % S | | 13,55 % S |

Beispiel 9: Lösungsmittelfreie Herstellung von 2,6-Bis(n-octylthiomethyl)-4-t-butylphenol

Man geht wie im Beispiel 4 vor, jedoch unter Verwendung folgenden Gemisches: 240,3 g (1,6 Mol) 4-t-Butylphenol, 468,2 g (3,2 Mol) Paraformaldehyd und 100,9 g (3,32 Mol) n-Octanthiol. Anschliessend wird 8,7 g (0,19 Mol) Dimethylamin-Gas in die Suspension eingeleitet. Die Reaktionszeit beträgt 3 Stunden. Man erhält 670 g (90 % der Theorie) 2,6-Bis(n-octylthiomethyl)-4-t-butylphenol mit einer Reinheit von 94,1 % (absolute Ausbeute: 84,7 %).

Beispiel 10: Herstellung von 2,2-Bis[4,4'-dihydroxy-3,3',5,5'-tetrakis(n-octylthiomethyl)phenyl]-propan

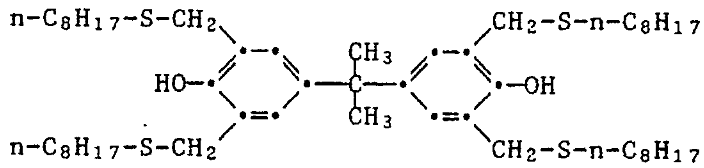

Man geht wie im Beispiel 8 vor, jedoch unter Verwendung folgenden Gemisches: 23,3 g (0,10 Mol) Bisphenol A, 20,4 g (0,68 Mol) Paraformaldehyd, 60,33 g (0,41 Mol) n-Octanthiol, 7,5 g 33 %-iges ethanolisches Dimethylamin (entspricht etwa 0,056 Mol Dimethylamin) und 40 ml (38 g) N,N-Dimethylformamid. Die Reaktionszeit beträgt 6 Stunden. Man erhält 86,4 g (99 % der Theorie) des Produkts als leicht gelbliches Oel mit einer Reinheit von 94,6 % (absolute Ausbeute: 93,7 %).

Analysewerte:

| | | | |
|---|---|---|---|
| Berechnet: | 71,10 % C | Gefunden: | 70,87 % C |
| | 10,30 % H | | 10,51 % H |
| | 14,89 % S | | 14,87 % S |

Beispiel 11: Herstellung von 2,4-Bis(n-octylthiomethyl)-6-methyl-phenol

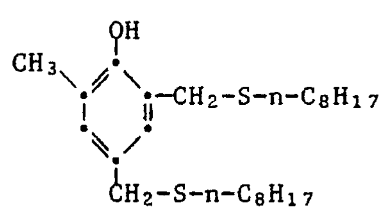

Man geht wie in Beispiel 1 vor, jedoch unter Verwendung von 0,93 g (0,03 Mol) Methylamin als Base, anstelle von 0,03 Mol Dimethylamin. Man erhält 60,38 g (98,5 % der Theorie) des Produktes als eine gelbliche Flüssigkeit mit einer Reinheit von 78,3 % (absolute Ausbeute: 77,1 %).

## Patentansprüche

**1.** Verfahren zur Herstellung von Verbindungen der Formeln I oder II

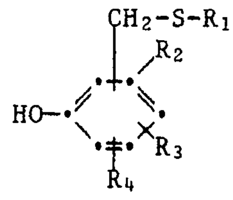
(I)

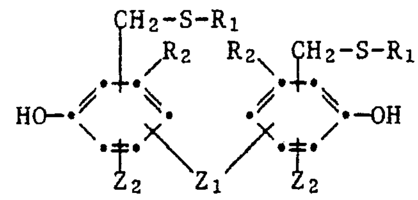
(II)

,

worin
$R_1$ $C_1$-$C_{20}$-Alkyl bedeutet, welches unsubstituiert ist oder durch 1 oder 2 Hydroxylgruppen substituiert oder durch -O- unterbrochen ist, oder $C_1$-$C_4$-Alkylen-$COOR_5$, $C_1$-$C_4$-Alkylen-CO-$NR_5R_6$, $C_5$-$C_{12}$-Cycloalkyl, Phenyl, 1-Naphthyl, 2-Naphthyl, $C_1$-$C_4$-Alkyl-Phenyl oder Phenyl-$C_1$-$C_4$-Alkyl bedeutet,
$R_2$ Wasserstoff, $C_1$-$C_{20}$-Alkyl, $C_2$-$C_{18}$-Alkenyl oder Halogen ist,
$R_3$ und $R_4$ unabhängig voneinander $C_1$-$C_{20}$-Alkyl, Allyl, $C_5$-$C_{12}$-Cycloalkyl, Phenyl, Phenyl-$C_1$-$C_4$-Alkyl, Halogen oder -$CH_2$-S-$R_1$ bedeuten, mit der Massgabe, dass mindestens eines von $R_3$ und $R_4$ für -$CH_2$-S-$R_1$ steht,
$R_5$ $C_1$-$C_{20}$-Alkyl, Allyl, $C_5$-$C_{12}$-Cycloalkyl, Phenyl oder Benzyl bedeutet,
$R_6$ Wasserstoff, $C_1$-$C_{20}$-Alkyl oder $C_2$-$C_{18}$-Alkenyl ist,
$Z_1$ für -S- oder -C($Z_3$)($Z_4$)- steht,
$Z_2$ Wasserstoff, $C_1$-$C_{20}$-Alkyl oder -$CH_2$-S-$R_1$ ist,
$Z_3$ Wasserstoff oder Methyl bedeutet und $Z_4$ Wasserstoff oder $C_1$-$C_8$-Alkyl ist, mit der Massgabe, dass die Phenole der Formeln I und II in m-Stellung frei von der funktionellen Gruppe -$CH_2$-S-$R_1$ sind, durch Umsetzung eines Phenols der Formeln III oder IV

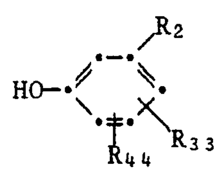
(III)

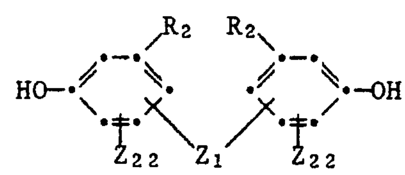
(IV)

,

worin
$R_2$ und $Z_1$ die zuvor genannte Bedeutung haben,
$R_{33}$ und $R_{44}$ unabhängig voneinander Wasserstoff, $C_1$-$C_{20}$-Alkyl, Allyl, $C_5$-$C_{12}$-Cycloalkyl, Phenyl, Phenyl-$C_1$-$C_4$-Alkyl oder Halogen bedeuten, mit der Massgabe, dass mindestens eines von $R_{33}$ und $R_{44}$ für Wasserstoff steht, und
$Z_{22}$ Wasserstoff oder $C_1$-$C_{20}$-Alkyl ist,
mit Formaldehyd oder einer unter den Reaktionsbedingungen Formaldehyd freisetzenden Verbindung und mit mindestens einem Merkaptan $R_1$-SH in Gegenwart einer Base, dadurch gekennzeichnet, dass die Base Mono-, Di-oder Trimethylamin oder Mono- oder Diethylamin ist.

**2.** Verfahren nach Anspruch 1, dadurch gekennzeichnet, dass die Base Mono- oder Dimethylamin ist.

**3.** Verfahren nach Anspruch 2, dadurch gekennzeichnet, dass die Base Dimethylamin ist.

**4.** Verfahren nach Anspruch 1, dadurch gekennzeichnet, dass man 1-50 Mol% der Base, bezogen auf das

Merkaptan, einsetzt.

5. Verfahren nach Anspruch 1, dadurch gekennzeichnet, dass die Umsetzung in Abwesenheit eines Lösungsmittels erfolgt.

6. Verfahren nach Anspruch 1 zur Herstellung von Verbindungen der Formel

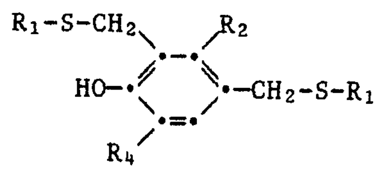

worin $R_1$, $R_2$ und $R_4$ die in Anspruch 1 genannte Bedeutung haben.

7. Verfahren nach Anspruch 6, worin $R_2$ Wasserstoff oder Methyl bedeutet und $R_4$ Methyl, t-Butyl oder Cyclohexyl ist.

8. Verfahren zur Herstellung von Verbindungen der Formel I nach Anspruch 1, worin $R_1$ $C_8$-$C_{12}$-Alkyl, -$CH_2$-COO-Alkyl mit 1 bis 18 C-Atomen im Alkylrest, -$CH_2$-$CH_2$-OH, Phenyl oder Benzyl bedeutet.

9. Verfahren nach Anspruch 8, worin $R_1$ $C_8$-$C_{12}$-Alkyl bedeutet.

10. Verfahren nach Anspruch 8, worin $R_1$ -$CH_2$-COO-$CH_2$-C($C_2H_5$)-$(CH_2)_3$-$CH_3$ ist.

## Claims

1. A process for the preparation of a compound of formula I or II

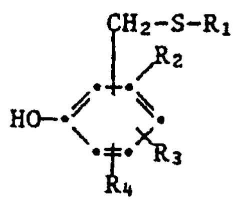

(I)

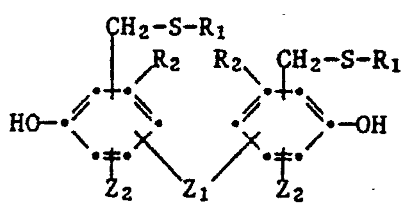

(II)

,

in which
$R_1$ is $C_1$-$C_{20}$alkyl which is unsubstituted or substituted by 1 or 2 hydroxyl groups or interrupted by -O-, or is $C_1$-$C_4$alkylene-COOR$_5$, $C_1$-$C_4$alkylene-CO-NR$_5$R$_6$, $C_5$-$C_{12}$cycloalkyl, phenyl, 1-naphthyl, 2-naphthyl, $C_1$-$C_4$alkylphenyl or phenyl-$C_1$-$C_4$alkyl,
$R_2$ is hydrogen, $C_1$-$C_{20}$alkyl, $C_2$-$C_{18}$alkenyl or halogen,
$R_3$ and $R_4$ are each independently of the other $C_1$-$C_{20}$alkyl, allyl, $C_5$-$C_{12}$cycloalkyl, phenyl, phenyl-$C_1$-$C_4$alkyl, halogen or -$CH_2$-S-$R_1$, with the proviso that at least one of $R_3$ and $R_4$ is -$CH_2$-S-$R_1$,
$R_5$ is $C_1$-$C_{20}$alkyl, allyl, $C_5$-$C_{12}$cycloalkyl, phenyl or benzyl,
$R_6$ is hydrogen, $C_1$-$C_{20}$alkyl or $C_2$-$C_{18}$alkenyl,
$Z_1$ is -S- or -C($Z_3$)($Z_4$)-,
$Z_2$ is hydrogen, $C_1$-$C_{20}$alkyl or -$CH_2$-S-$R_1$,
$Z_3$ is hydrogen or methyl and $Z_4$ is hydrogen or $C_1$-$C_8$alkyl, with the proviso that the phenol of formula I or II does not contain the functional group -$CH_2$-S-$R_1$ in the m-position,
by reaction of a phenol of formula III or IV

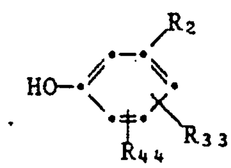

(III)

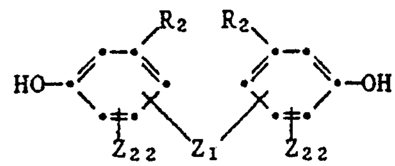

(IV)

in which
$R_2$ and $Z_1$ are as defined above,
$R_{33}$ and $R_{44}$ are each independently of the other hydrogen, $C_1$-$C_{20}$alkyl, allyl, $C_5$-$C_{12}$cycloalkyl, phenyl, phenyl-$C_1$-$C_4$alkyl or halogen, with the proviso that at least one of $R_{33}$ and $R_{44}$ is hydrogen, and
$Z_{22}$ is hydrogen or $C_1$-$C_{20}$alkyl,
with formaldehyde or a compound that liberates formaldehyde under the reaction conditions and with at least one mercaptan $R_1$-SH, in the presence of a base, wherein the base is mono-, di- or trimethylamine or mono- or diethylamine.

**2.** A process according to claim 1, wherein the base is monomethylamine or dimethylamine.

**3.** A process according to claim 2, wherein the base is dimethylamine.

**4.** A process according to claim 1, wherein 1 to 50 mol% of the base is used, based on the mercaptan.

**5.** A process according to claim 1, wherein the reaction is carried out in the absence of a solvent.

**6.** A process according to claim 1 for the preparation of a compound of formula

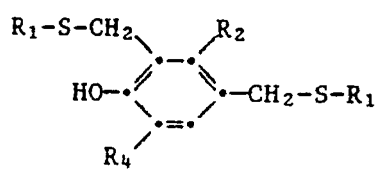

in which $R_1$, $R_2$ and $R_4$ are as defined in claim 1.

**7.** A process according to claim 6, in which $R_2$ is hydrogen or methyl and $R_4$ is methyl, tert-butyl or cyclohexyl.

**8.** A process for the preparation of a compound of formula I according to claim 1, in which $R_1$ is $C_8$-$C_{12}$alkyl, $-CH_2-COO-$alkyl containing 1 to 18 carbon atoms in the alkyl moiety, $-CH_2-CH_2-OH$, phenyl or benzyl.

**9.** A process according to claim 8, in which $R_1$ is $C_8$-$C_{12}$alkyl.

**10.** A process according to claim 8, in which $R_1$ is $-CH_2-COO-CH_2-C(C_2H_5)-(CH_2)_3-CH_3$.

## Revendications

**1.** Procédé pour préparer des composés répondant à l'une des formules I et II :

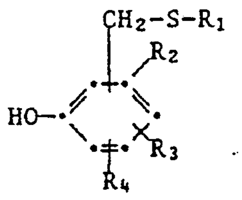

(I)

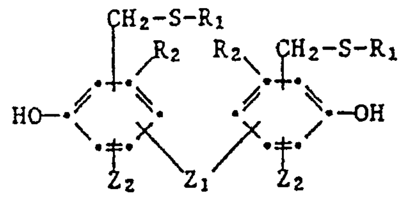

(II)

dans lesquelles

- $R_1$ représente un alkyle en $C_1$-$C_{20}$ qui ne porte pas de substituant ou porte un ou deux radicaux hydroxy ou est interrompu par -O-, ou représente un radical $C_1$-$C_4$-alkylène-$COOR_5$, $C_1$-$C_4$-alkylène-CO-$NR_5R_6$, $C_5$-$C_{12}$-cycloalkyle, phényle, naphtyle-1, naphtyle2, $C_1$-$C_4$-alkyl-phényle ou phényl-$C_1$-$C_4$-alkyle,
- $R_2$ représente l'hydrogène, un alkyle en $C_1$-$C_{20}$, un alcényle en $C_2$-$C_{18}$ ou un halogène,
- $R_3$ et $R_4$ représentent chacun, indépendamment l'un de l'autre, un alkyle en $C_1$-$C_{20}$, un allyle, un cycloalkyle en $C_5$-$c_{12}$, un phényle, un phényl-$C_1$-$C_4$-alkyle, un halogène ou un radical -$CH_2$-S-$R_1$, avec la condition qu'au moins un des symboles $R_3$ et $R_4$ représente un radical -$CH_2$-S-$R_1$,
- $R_5$ représente un alkyle en $C_1$-$C_{20}$, un allyle, un cycloalkyle en $C_5$-$C_{12}$, un phényle ou un benzyle,
- $R_6$ représente l'hydrogène, un alkyle en $C_1$-$C_{20}$ ou un alcényle en $C_2$-$C_{18}$,
- $Z_1$ représente -S- ou -C($Z_3$) ($Z_4$)-,
- $Z_2$ représente l'hydrogène, un alkyle en $C_1$-$C_{20}$ ou un radical -$CH_2$-S-$R_1$,
- $Z_3$ représente l'hydrogène ou un méthyle et
- $Z_4$ représente l'hydrogène ou un alkyle en $C_1$-$C_8$,

avec la restriction que les phénols de formules I et II ne portent pas de radical fonctionnel -$CH_2$-S-$R_1$ à la position métha,

par réaction d'un phénol répondant à l'une des formules III et IV :

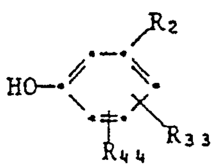

(III)

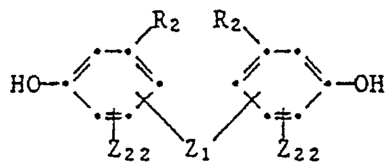

(IV)

dans lesquelles

- $R_2$ et $Z_1$ ont les significations qui leur ont été données ci-dessus,
- $R_{33}$ et $R_{44}$ représentent chacun, indépendamment l'un de l'autre, l'hydrogène, un alkyle en $C_1$-$C_{20}$, un allyle, un cycloalkyle en $C_5$-$C_{12}$, un phényle, un phényl-$C_1$-$C_4$-alkyle ou un halogène, avec la condition qu'au moins un des symboles $R_{33}$ et $R_{44}$ représente l'hydrogène, et
- $Z_{22}$ représente l'hydrogène ou un alkyle en $C_1$-$C_{20}$,

avec le formaldéhyde ou un composé capable de libérer du formaldéhyde dans les conditions de la réaction et avec au moins un mercaptan $R_1$-SH, en présence d'une base, procédé caractérisé en ce que la base est la mono-, la di- ou la triméthylamine, ou la mono- ou la diéthylamine.

2. Procédé selon la revendication 1 caractérisé en ce que la base est la monométhylamine ou la diméthylamine.

3. Procédé selon la revendication 2 caractérisé en ce que la base est la diméthylamine.

4. Procédé selon la revendication 1 caractérisé en ce qu'on utilise de 1 à 50 % en moles de la base par rapport au mercaptan.

**5.** Procédé selon la revendication 1 caractérisé en ce que la réaction est effectuée sans solvant.

**6.** Procédé selon la revendication 1 pour la préparation de composés répondant à la formule :

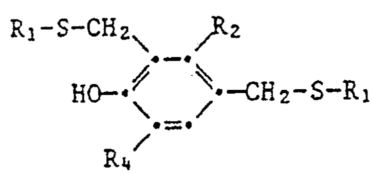

dans laquelle $R_1$, $R_2$ et $R_4$ ont les significations qui leur ont été données à la revendication 1.

**7.** Procédé selon la revendication 6 dans lequel $R_2$ représente l'hydrogène ou un radical méthyle et $R_4$ un radical méthyle, tert-butyle ou cyclohexyle.

**8.** Procédé pour préparer des composés de formule I selon la revendication 1, dans lequel $R_1$ représente un alkyle en $C_8$-$C_{12}$, un radical $-CH_2-COO-$alkyl contenant de 1 à 18 atomes de carbone dans sa partie alkyle, un radical $-CH_2-CH_2-OH$, un phényle ou un benzyle.

**9.** Procédé selon la revendication 8 dans lequel $R_1$ représente un alkyle en $C_8$-$C_{12}$.

**10.** Procédé selon la revendication 8 dans lequel $R_1$ représente un radical $-CH_2-COO-CH_2-CH(C_2H_5)-(CH_2)_3-CH_3$.